# DEMANDE DE BREVET EUROPEEN

(11) **EP 2 165 666 A1**
(43) Date de publication de la demande: **24.03.2010**
(21) Numéro de dépôt: 09305814.7
(22) Date de dépôt: 07.09.2009
(51) Int. Cl.: A61B 17/28, A61B 17/00

(54) **Pince pour prostatectomie**

(30) Priorité: 17.09.2008 FR 0856239
(71) Demandeur: Aspide Medical, 42350 La Talaudiere (FR); Selarl Maire Jerome - Roumier Xavier - Centre d'Urologie de la Parisiere, 26300 Bourg de Peage (FR)
(72) Inventeur: Maire, Jérôme, 26100, ROMANS (FR); Roumier, Xavier, 26260, SAINT-DONAT (FR)
(74) Mandataire: Dupuis, François

(57) **Abrégé**

Cette pince est remarquable ce qu'elle comprend un corps (11) situé en partie arrière se prolongeant axialement et dans un même plan par un axe de centrage (12) fixe de grande longueur susceptible de pénétrer dans le canal de l'urètre (U), et en ce que le corps est aménagé avec une échancrure (11a) pour le pivotement d'une branche (13) de configuration longitudinale curviligne orientée vers ledit axe de centrage et en section transversale en forme de gouttière (13a) dans toute la longueur de la branche, ladite branche curviligne étant susceptible de serrer le demi-volume extérieur de la prostate (P) pour assurer son maintien et sa tenue.

## Description

L'invention se rattache au secteur technique des appareils et instruments utilisés en chirurgie et en particulier aux pinces de prostatectomie utilisées lors du traitement du cancer de la prostate en vue de l'ablation de celle-ci.

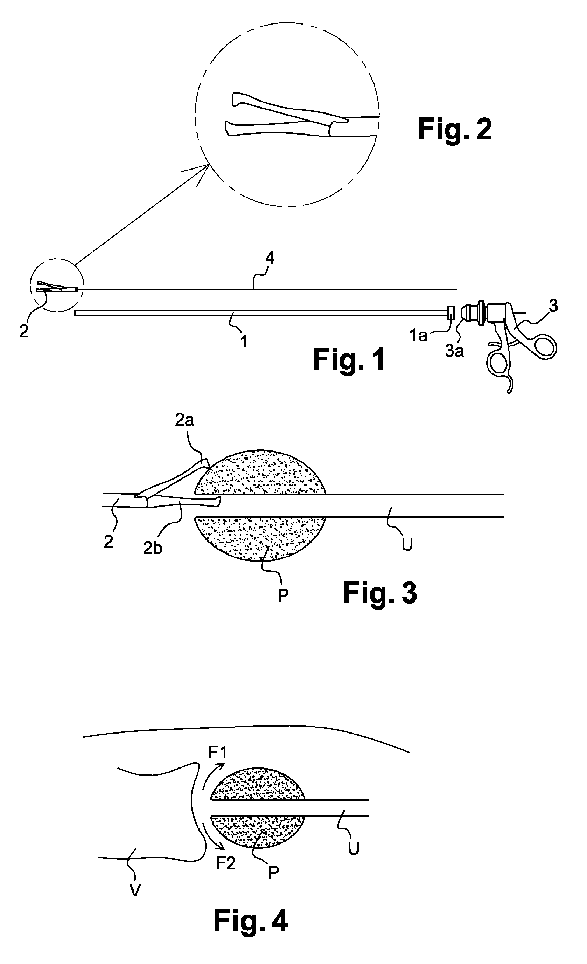

Les instruments utilisés à cet effet sont de type de ceux utilisés en coelioscopie et un exemple est illustré figure 1 des dessins. L'instrument concerné est ainsi réalisé en trois parties, un tube (1), une pince (2) et une poignée de commande (3). La pince (2) comprend ainsi une partie tubulaire (4) de grande longueur qui est enfilée dans le tube. Ce dernier présente à son extrémité arrière une bague (1a) vissée sur la buse (3a) de la poignée de commande. La partie pince (2) comprend en extrémité deux branches (2a - 2b) formant mâchoires dont l'ouverture-fermeture est commandée par la poignée de commande. On a représenté, figure 2 l'art antérieur de la pince prise en tant que telle utilisée en coelioscopie du type de celle connue dans le commerce sous la référence «BABCOCK» de la société DUCHATEAU. Les deux branches (2a-2b) de la pince sont articulées l'une par rapport à l'autre et présentent chacune en bout une forme en crochet qui est susceptible de venir s'agripper à la paroi en regard de la prostate. On a ainsi représenté figure 3, de manière schématique, l'utilisation de ce type de pince de prostatectomie dans son environnement d'utilisation.

On a représenté par P la prostate, par V la vessie, par U le canal de l'urètre. On a schématisé, figure 4, l'instrument sous forme de pince avec les deux flèches (F1-F2) représentant ses branches qui viennent s'engager et pincer la prostate. Un trocart est généralement utilisé pour le passage du tube de l'instrument.

Le problème posé réside en ce que les pinces existants sur le marché et en particulier celle référencée « BABCOCK » illustrée figure 2 sont trop petites, ont une géométrie inadaptée et n'ont pas une prise suffisante malgré leurs extrémités en forme de crochets. L'ablation de la prostate requiert à travers les tissus environnants de manipuler celle-ci par des mouvements en rotation, en va et vient, en vue de la séparer de la vessie, en sectionnant le canal de l'urètre. La présence en outre d'un outil de sectionnement autour ou près de l'instrument constitue une gêne et facilite par les différentes manipulations une mauvaise tenue voir perte de prise de la pince de l'instrument sur la prostate. Il est possible également d'utiliser ce type de pince comme représenté figure 3 avec une branche qui pénètre dans le canal de l'urètre après dégagement de celui-ci, tandis que l'autre branche vient s'accrocher sur la partie en regard de la paroi de la prostate. Compte tenu de la configuration terminale en crochet des branches de la pince, cela entraine un détérioration de la partie de l'urètre et prostate. Même si la prostate est destinée à être enlevée, (le chirurgien raccorde les parties de conduit de l'urètre situées de part et d'autre de la prostate par des sutures), on constate une détérioration de la prostate, ce qui est nuisible à la qualité des examens qui sont effectués sur celle-ci pour des travaux d'analyse et de recherches complémentaires (...). En outre, on est toujours confronté au problème de la prise externe de la prostate tel qu'évoqué précédemment.

Ainsi, les moyens utilisés selon l'art antérieur sont peu satisfaisants en terme de qualité, de tenue, et de prise de la prostate avec une détérioration réelle engendrée sur la prostate elle-même, qui est atteinte et qui doit être enlevée et analysée.

La démarche du demandeur a donc été de réfléchir et de trouver une solution apte à faciliter la prise et la tenue de la prostate pour faciliter son ablation sans pour autant la détériorer en plus des séquelles inhérentes à la tumeur.

La solution apportée par le demandeur répond de manière satisfaisante à ce problème posé. Cette solution concerne exclusivement la partie pince, les autres moyens du type tube, poignée de commande d'ouverture-fermeture de la pince étant du type de ceux connus.

Selon une première caractéristique de l'invention, la pince de prostatectomie du type comprenant des branches articulées l'une par rapport à l'autre et présentant une partie ancrée destinée à être fixée au tube de liaison, lui-même associé à une poignée de commande est remarquable en ce qu'elle comprend un corps situé en partie arrière se prolongeant axialement et dans un même plan par un axe de centrage fixe de grande longueur susceptible de pénétrer dans le canal de l'urètre, et en ce que le corps est aménagé avec une échancrure pour le pivotement d'une branche de configuration longitudinale curviligne orientée vers ledit axe de centrage et en section transversale en forme de gouttière dans toute la longueur de la branche, ladite branche curviligne étant susceptible d'entourer plus de la moitié du demi-volume extérieur de la prostate pour assurer son maintien et sa tenue.

Ces caractéristiques et d'autres encore ressortiront bien de la suite de la description.

Pour fixer l'objet de l'invention illustré de manière non limitative aux figures des dessins où :
- La figure 1 est une vue d'un instrument utilisé en prostactomie selon l'art antérieur avec une pince, un tube de liaison et une poignée de commande.
- La figure 2 est une vue d'une pince de prostactomie du type « BABCOCK » selon l'art antérieur.
- La figure 3 est une vue à caractère schématique illustrant la position de la pince par rapport à la prostate selon l'art antérieur.
- La figure 4 est une vue de la pince selon la figure 2 introduite dans le canal de l'urètre et sur la paroi en regard de la prostate.

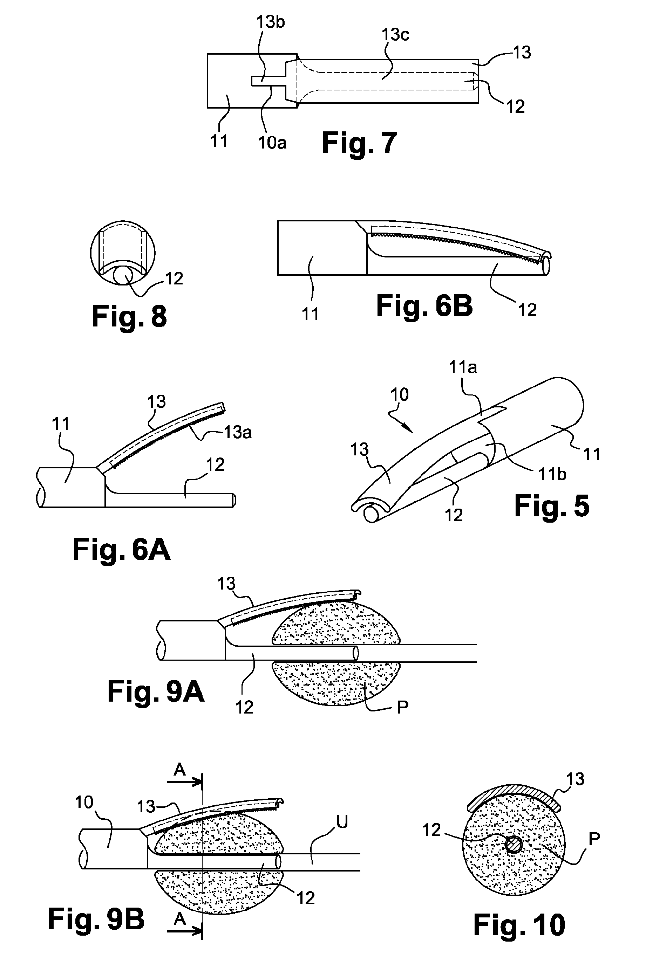

- La figure 5 est une vue en perspective de la pince de prostatectomie selon l'invention en position fermée de non utilisation.
- La figure 6A-6B sont des vues de côté de la pince selon la figure 5 en position ouverte et fermée.
- La figure 7 est une vue de dessus de la pince selon la figure 5.
- La figure 8 est une vue de face de la pince selon la figure 5.
- Les figures 9A, 9B sont des vues à caractère schématique illustrant la position de la pince selon l'invention en vue de l'ablation de la prostate en situation pénétrant dans le canal de l'urètre, et entourant et maintenant la prostate sans détérioration.
- La figure 10 est une vue en coupe selon la ligne A-A de la figure 9.

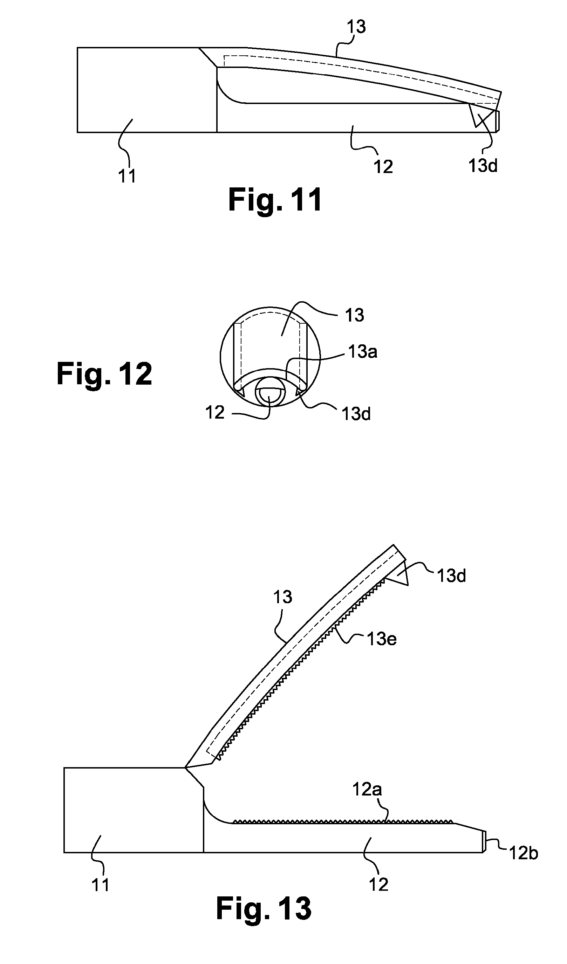

- La figure 11 illustre la pince présentant, en extrémité de sa branche, des griffes.
- La figure 12 est une vue partielle de face de l'extrémité de la pince.
- La figure 13 est une vue en variante de la figure 11, l'axe de centrage et la branche articulée présentant des saillies.

Afin de rendre plus concret l'objet de l'invention, on le décrit maintenant d'une manière non limitative illustrée aux figures des dessins.

La pince de prostactomie selon l'invention est référencée dans son ensemble par (10). Elle présente un corps de base (11) situé en partie arrière qui est agencé pour être solidarisé de manière démontable à un tube relié à la poignée de commande. Ledit corps de base (11) est de configuration cylindrique et allongée et présente dans sa partie avant un axe de centrage (12) tubulaire destiné à être introduit et positionné dans l'urètre (U) se trouvant entourée par la prostate (P). Cet axe de centrage (12) de grande longueur étant déporté sur une large distance dans le canal de l'urètre (U) par rapport au corps tout en étant dans l'alignement de celui-ci. Cet axe de centrage est fixé et peut être rapporté audit corps.

Dans sa partie supérieure, le corps présente une échancrure (11a) formant fente débouchant à l'avant dudit corps et autorisant la fixation et l'articulation par pivotement d'une branche (13) de configuration longitudinale curviligne orientée vers ledit axe de centrage et en section transversale en forme de gouttière (13a) dans toute la longueur de la branche. Cette forme en gouttière peut être ajourée par une lumière (13c) (fenêtre). Ladite branche présente ainsi un talon (13b) pénétrant dans l'échancrure précitée, un axe transversal assurant la liaison et l'articulation. La commande de l'articulation de ladite branche s'effectue par un moyen de renvoi classique non représenté intégré dans ledit corps de la pince et relié de manière classique par le tube à la poignée de commande selon la pratique connue en coelioscopie. Ainsi qu'il apparaît aux dessins, la section transversale de la branche forme un profil courbe en gouttière ou est ajourée par une lumière (13c) (fenêtre), sans aspérité, ni forme crochue ou dentelée permettant un appui ferme, et sur une grande longueur sur la prostate sans la détériorer. De manière préférentielle, la branche (13) est aménagée à son extrémité avant avec deux griffes (13d) d'ancrage. L'axe de centrage (12) et la branche sont agencés avec des saillies intérieures en regard (12a) (13e) pour compléter le maintien sur la prostate. L'extrémité (12b) de l'axe de centrage peut être en biseau.

La longueur de cette branche articulée est telle qu'elle-vient coiffer très largement la partie en regard de la prostate et en particulier selon une distance telle qu'elle recouvre celle-ci au-delà de sa partie médiane, selon un faisceau continu.

La branche articulée se trouve dans un plan au-dessus de l'axe de centrage et les deux ont sensiblement la même longueur hors face avant (11b) du corps.

Selon une disposition avantageuse de l'invention, la partie d'articulation de la branche se trouve en retrait de la face transversale (11b) avant du corps de sorte à créer une large amplitude de mouvement et permettre une introduction optimum de l'axe de maintien dans le canal de l'urètre. Par ailleurs, la partie d'articulation elle-même est protégée et n'est pas en contact avec la prostate et le canal de l'urètre.

L'écart entre l'axe de centrage (12) et la branche (13) à l'endroit ou ils sont reliés au corps de base (11) permet d'optimiser le serrage.

L'axe de centrage (12) peut être plein ou creux. Sa longueur est adaptée à la taille de la prostate.

La pince selon l'invention est montée sur un support pouvant être introduit par un trocart classique de chirurgie.

Les avantages ressortent bien de l'invention. On souligne tout d'abord la meilleure prise et tenue de la prostate due à l'axe pénétrant dans le canal de l'urètre et la forme particulière de la branche formant gouttière ou ajourée par une lumière (fenêtre) qui recouvre une large partie de la prostate sans la détériorer, l'ensemble permettant et facilitant les manipulations et mouvements sur la prostate en vue de son ablation.

Un autre avantage réside dans la meilleure tenue et prise de la prostate qui est maintenue en particulier par un large faisceau longitudinal dans la partie intérieure de la branche.

Un autre avantage réside dans la simplicité de la pince qui est réalisée en matériau classique compatible à son environnement d'utilisation.

Les pinces de prostatectomie selon l'invention sont adaptées selon les tailles des prostates des individus concernés.

## Revendications

1. Pince de prostatectomie du type comprenant des branches articulées l'une par rapport à l'autre et présentant une partie ancrée destinée à être fixée au tube de liaison, lui-même associé à une poignée de commande est remarquable en ce qu'elle comprend un corps (11) situé en partie arrière se prolongeant axialement et dans un même plan par un axe de centrage (12) fixe de grande longueur susceptible de pénétrer dans le canal de l'urètre (U), et en ce que le corps est aménagé avec une échancrure (11a) pour le pivotement d'une branche (13) de configuration longitudinale curviligne orientée vers ledit axe de centrage et en section transversale en forme de gouttière (13a) dans toute la longueur de la branche, ladite branche curviligne étant susceptible de serrer le demi-volume extérieur de la prostate (P) pour assurer son maintien et sa tenue.

2. Pince selon la revendication 1, **caractérisée en ce que** l'axe de centrage (12) de grande longueur est déporté sur une large distance dans le canal de l'urètre (U) par rapport au corps tout en étant dans l'alignement de celui-ci.

3. Pince selon la revendication 2, **caractérisée en ce que** l'axe de centrage est fixé et peut être rapporté audit corps.

4. Pince selon la revendication 1, **caractérisée en ce que** dans sa partie supérieure, le corps présente une échancrure (11a) formant fente débouchant à l'avant dudit corps et autorisant la fixation et l'articulation par pivotement d'une branche (13) de configuration longitudinale curviligne et en section transversale en forme de gouttière (13a) ou de configuration longitudinale curviligne et ajourée par une lumière (fenêtre), et **en ce que** ladite branche présente ainsi un talon (13b) pénétrant dans l'échancrure précitée, un axe transversal assurant la liaison et l'articulation.

5. Pince selon la revendication 4 **caractérisée en ce que** la commande de l'articulation de ladite branche (13) s'effectue par un moyen de renvoi intégré dans ledit corps de la pince et relié de manière classique par le tube à la poignée de commande

6. Pince selon la revendication 1, **caractérisée en ce que** la section transversale de la branche forme un profil courbe en gouttière ou est ajourée par une lumière (fenêtre), sans aspérité, ni forme crochue ou dentelée permettant un appui ferme, et sur une grande longueur sur la prostate sans la détériorer.

7. Pince selon la revendication 1, **caractérisée en ce que** la branche articulée se trouve dans un plan au-dessus de l'axe de centrage et décalé pour optimiser le serrage et les deux ont sensiblement la même longueur hors face avant (11b) du corps.

8. Pince selon la revendication 1, **caractérisée en ce que** la partie d'articulation de la branche se trouve en retrait de la face transversale (11b) avant du corps de sorte à créer une large amplitude de mouvement et permettre une introduction optimum de l'axe de maintien dans le canal de l'urètre.

9. Pince, selon la revendication 1, **caractérisée en ce que** la branche (13) est aménagée à son extrémité avant avec deux griffes d'ancrage (13d).

10. Pince, selon la revendication 1, **caractérisée en ce que** l'axe de centrage (12) et la branche (13) sont aménagés avec des saillies intérieures en regard (12a) (13e).
